# EUROPEAN PATENT APPLICATION

(11) **EP 1 929 869 A1**
(43) Date of publication of application: **11.06.2008**
(21) Application number: 06798395.7
(22) Date of filing: 28.09.2006
(51) Int. Cl.: A01N 43/72, A01N 63/00, A01N 63/02, A01P 3/00, C07K 7/02

(54) **COMPOSITION FOR CONTROLLING COMMON SCAB OF AGRICULTURAL CROP CONTAINING SURFACTIN**

(30) Priority: 30.09.2005 JP 2005287922
(71) Applicant: SUNTORY LIMITED, Osaka-shi, Osaka 530-8203 (JP)
(72) Inventor: SAEKI, Takeshi, Hyogo 662-0046 (JP); KOMURA, Hajime, Osaka 618-0024 (JP); OHWADA, Takuji, Hokkaido 080-0027 (JP); KINOSHITA, Mikio, Hokkaido 080-0027 (JP)
(74) Representative: Vossius & Partner
(86) International application number: PCT/JP2006/319255
(87) International publication number: WO 2007/040131

(57) **Abstract**

The present invention relates to a composition for controlling scab disease in agricultural products, which comprises a compound of Formula I or a salt thereof. The present invention further relates to a method for controlling scab disease in agricultural products by using a composition for controlling scab disease, which comprises a compound of Formula I or a salt thereof.

## Description

### TECHNICAL FIELD

The present invention relates to a composition for controlling scab disease in agricultural products, which comprises a compound of Formula I or a salt thereof. The present invention further relates to a method for controlling scab disease in agricultural products by using a composition for controlling scab disease, which comprises a compound of Formula I or a salt thereof.

### BACKGROUND ART

Potato scab, which is one of the difficult-to-control soil diseases caused by actinomycetes of the genus *Streptomyces,* has been found to occur at many places in the world since a long time ago. Diseased potatoes develop red-brown scab-like lesions on their tuber surfaces, and tissues of the lesions become corked to generate small protrusions and/or small cracks. As a result, such diseased potatoes are subject to significant loss in their commercial value for either fresh or processing; and also suffer a great reduction in their starch value when the disease is severe. In the Hokkaido prefecture of Japan, increasing damage after the 1980s due to spread of areas with diseased soil has constituted a major obstacle in maintaining a stable supply of potatoes. Under these circumstances, studies on potato scab have been pursued.

Control measures previously used against potato scab include disinfection of seed potatoes with an antibiotic (e.g., streptomycin), as well as sterilization/disinfection of the soil using a synthetic drug (e.g., chloropicrin or pentachloronitrobenzene).

However, since the main source of potato scab infection is the soil, no significant improvement is seen when seed potatoes are simply disinfected as in the former case. On the other hand, soil sterilization with a synthetic drug as in the latter case may have a disadvantage that it also kills soil microorganisms having the ability to antagonize and repress infectious pathogens in the soil, and thereby conversely facilitating occurrence of disease. Moreover, these control measures have other serious problems responsible for environmental destruction and/or occurrence of industrial accidents due to drug-induced suffering, such as the risk of causing accumulation of harmful chemical substances in the soil and/or impairing the health of workers who spray the drugs.

Another control measure aims to decrease the soil pH to avoid a pH range suitable for growth of *Streptomyces* spp., which are pathogens of scab disease. However, this measure has very limited applications, for example because the yield is greatly reduced and agricultural products to be cultivated after harvesting of potatoes are adversely affected.

As a control measure against plant diseases which is free from problems such as those mentioned above, a biological control technique using microorganisms has been developed and many reports have been issued for this technique. However, there are few cases of biological control being used against potato scab, as exemplified by a technique in which seed potatoes are soaked in a suspension containing at least one of *Pseudomonas fluorescens* strain MD-4f, *Pseudomonas* sp. strain F13-1, *Enterobacter agglomerans* strain 2-3B and *Acinetobacter* sp. strain M24-1, or these bacteria are applied or sprayed in the form of dry powder or slurry over seed potatoes or the soil for potato cultivation (JP 01-193203 A); a technique in which plants such as potatoes are soaked in a suspension of *Bacillus* sp. strain KF-44 (JP 02-48509 A); a technique in which a material containing *Bacillus polymyxa* NT-107 and/or *Bacillus subtilis* NT-107 is sprayed directly over potatoes or sprayed over the soil for potato cultivation (JP 2001-327281 A); and a technique in which a material containing *Bacillus* strain MK-1-1 is sprayed over the soil for potato cultivation (JP 2004-2390 A).

However, for any of the above microbial techniques, none of the reports discloses the substance which actually has an antibacterial effect on the pathogen *Streptomyces* spp.

*Bacillus* subtilis-derived antibacterial compounds reported to date include cyclic peptide compounds which are generally well known, as exemplified by iturine (Peypoux et al. Biochemistry, 17, 3992-3996 (1978)) and surfactin (Vollenbroich et al. Appl. Environ. Microbiol., 63(1):44-9 (1997)). However, there is no knowledge about their antibacterial action against actinomycetes of the genus *Streptomyces,* which are pathogens of scab disease.

Likewise, JP 06-135811 A reports that iturine enhances its antibacterial action against plant pathogens when used in combination with surfactin, and that surfactin alone shows no antibacterial action. However, there is no information about their antibacterial action against actinomycetes of the genus *Streptomyces,* which are pathogens of scab disease.

### DISCLOSURE OF THE INVENTION

The present invention was made in consideration of the circumstances described above, and aims to provide a composition for controlling scab disease in agricultural products including potato, which is not only efficient and safe, but can also be used for actual field cultivation; as well as a method for controlling scab disease in agricultural products by using such a composition.

To solve the problems stated above, the inventors of the present invention have made extensive and intensive efforts to study components from *Bacillus subtilis* cultures. As a result, the inventors have found that a compound of Formula I or a salt thereof has antibacterial activity against actinomycetes of the genus *Streptomyces,* which are pathogens of scab disease in agricultural products, and have completed the present invention.

Thus, the present invention provides a composition for controlling scab disease in agricultural products, particularly a composition for controlling potato scab, which comprises a compound of Formula I or a salt thereof.

[Formula 1] 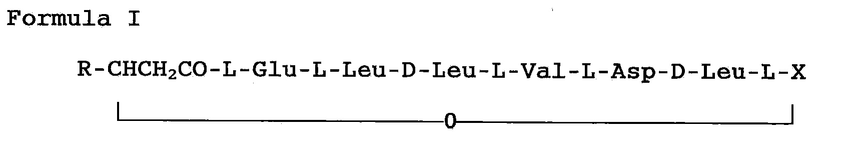 wherein R is C₁₁-C₁₄ alkyl, and X is selected from Leu, Val or Ile.

The present invention preferably provides a composition for controlling scab disease in agricultural products, which comprises surfactin (Formula II) or a salt thereof.

[Formula 2] 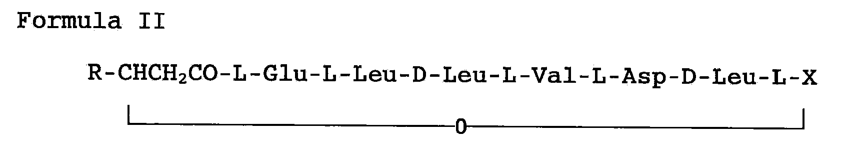 wherein R = (CH₂)₉-CH(CH₃)₂
X = Leu

The present invention provides such a composition for controlling scab disease, wherein the compound of Formula I or a salt thereof has a concentration of 1 to 10 mg/kg, as calculated on the basis of free form.

The present invention further provides a composition for controlling scab disease whose pathogen is an actinomycete of the genus *Streptomyces,* which comprises a compound of Formula I or a salt thereof. For example, the above actinomycete of the genus *Streptomyces* is *Streptomyces turgidiscabies.*

The present invention also provides a composition for controlling scab disease in agricultural products, which comprises a compound of Formula I or a salt thereof, wherein the composition further comprises cells of *Bacillus subtilis,* preferably cells of *Bacillus subtilis* strain SAM2267 (FERM P-17761).

The present invention provides a composition for controlling scab disease in agricultural products, which comprises a compound of Formula I or a salt thereof, wherein the composition comprises zeolite or an industrial food waste such as extracted coffee residue or extracted tea residue.

The present invention also provides a method for controlling scab disease in agricultural products by using a composition for controlling scab disease in agricultural products, which comprises a compound of Formula I or a salt thereof. By way of illustrative example, this control method involves contacting the above composition with seed potatoes, spraying the above control composition over the plant body, or alternatively, mixing or spraying the above control composition into/over the soil.

In the method for controlling scab disease in agricultural products which involves mixing or spraying the above control composition into/over the soil, a compound of Formula I or a salt thereof is mixed into or sprayed over the soil in an amount of 0.01 to 5 mg/m² per unit area of soil, as calculated on the basis of free form.

The present invention further provides the use of a compound of Formula I or a salt thereof for growth inhibition of actinomycetes of the genus *Streptomyces,* which are pathogens of scab disease in agricultural products including potato.

The present invention also provides a method for cultivating an agricultural product, which comprises using a control composition comprising a compound of Formula I or a salt thereof during cultivation of the agricultural product.

### BRIEF DESCRIPTION OF THE DRAWINGS

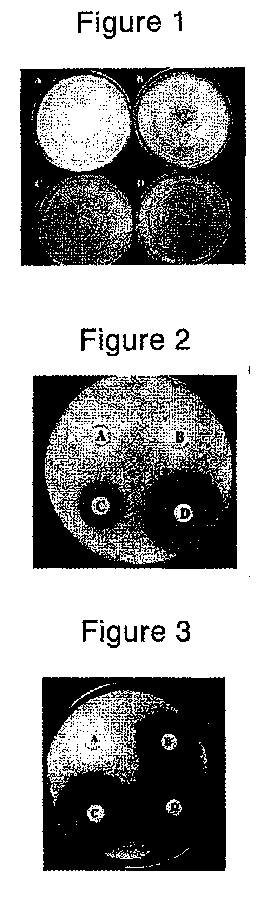

[Figure 1] Figure 1 shows the results examined for the effect of surfactin sodium concentrations in agar medium on the antibacterial action of surfactin sodium against a potato scab pathogen. Surfactin sodium concentrations in the medium was as follows: A: 0 µg/ml, B: 10 µg/ml, C: 50 µg/ml, and D: 100 µg/ml.
[Figure 2] Figure 2 shows the results examined, by the paper disk method, for the effect of surfactin concentrations on the antibacterial action of surfactin against a potato scab pathogen. The concentration of surfactin in methanol was set as follows: B: 0.01 mg/ml, C: 0.1 mg/ml, and D: 1.0 mg/ml. The liquid volume was set to 50 µl. Sample A was soaked with 50 µl methanol for use as a control.
[Figure 3] Figure 3 shows the results examined, by the paper disk method, for the effect of added surfactin volumes on the antibacterial action of surfactin against a potato scab pathogen. Paper disks were soaked with a 10 mg/ml methanol solution of surfactin in a volume of 10 µl (B), 50 µl (C) or 100 µl (D). Sample A was a surfactin-free disk.

### BEST MODE FOR CARRYING OUT THE INVENTION

### Composition for controlling scab disease in agricultural products

The term "agricultural product(s)" as used herein is intended to encompass all agricultural products which may suffer from scab disease, including beet, carrot, radish and potato.

The term "scab disease" as used herein refers to an infectious soil disease caused by actinomycetes. The disease develops scab-like lesions, which are categorized into recessed and protruded types depending on differences in environmental conditions, regardless of pathogen species.

The phrase "controlling scab disease" as used herein is intended to include preventing scab disease, keeping the occurrence of scab disease at a mild level and/or low frequency, and blocking the progress of scab disease, on the basis of the growth inhibitory effect on actinomycetes of the genus *Streptomyces,* which are pathogens of scab disease.

The composition of the present invention for controlling scab disease in agricultural products comprises a compound of Formula I or a salt thereof, preferably surfactin (Formula II) or a salt thereof.

It should be noted that R in Formula I is C₁₁-C₁₄ alkyl and the term "alkyl" refers to a linear saturated hydrocarbon or a branched saturated hydrocarbon. Namely, although the C₁₁-C₁₄ alkyl moiety includes those with a linear structure and those with different branched structures, preferred are those whose C₇ moiety adjacent to the cyclic structure has a linear structure, and more preferred are those whose C₉ moiety adjacent to the cyclic structure has a linear structure.

[Formula 3] 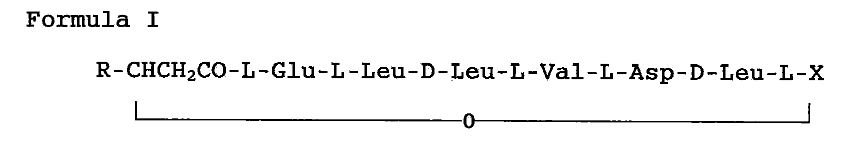 wherein R is C₁₁-C₁₄ alkyl, and X is selected from Leu, Val or Ile.

[Formula 4] 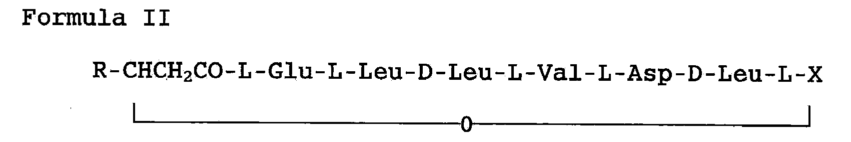 wherein R = (CH₂)₉-CH(CH₃)₂
X = Leu

Surfactin, which was found during extraction and separation of *Bacillus subtilis* cultures, has a molecular structure composed of a hydrophilic site formed by a 7-amino acid cyclic peptide and a hydrophobic site formed by a long fatty acid residue, as shown in the above formula. These amino acids comprise one molecule of L-glutamic acid, one molecule of L-aspartic acid, two molecules of D-leucine, and two molecules of L-leucine. The fatty acid moiety includes several members whose chain length and branched point(s) are different. Surfactin produces extremely low primary skin irritation and has good biodegradability. As is evident from the fact that surfactin is widely used as an ingredient in facial cleansers, shampoos and the like, it is a highly safe substance.

The compound of Formula I or a salt thereof used as an ingredient in the composition of the present invention for controlling scab disease in agricultural products may be any of these compounds or a combination thereof. Moreover, a salt of the compound of Formula I may be any salt such as an alkali metal or alkaline earth metal salt (e.g., sodium salt, potassium salt, calcium salt, magnesium salt), an amino acid salt (e.g., lysine salt), ammonium salt, an inorganic acid salt (e.g., hydrochloride salt, nitrate salt) or an organic acid salt (e.g., acetate salt, citrate salt), which may be in the form of a solvate such as a hydrate. Above all, sodium salt and free form are preferred for use.

Such a compound of Formula I or a salt thereof may be of any origin, such as microbial or synthetic origin, and also may be a commercially available product. In the case of microbial origin, it is possible to use a microbial fraction (e.g., cells or an extract thereof) containing a compound of Formula I or a salt thereof, although a purified product may be used.

To obtain a compound of Formula I or a salt thereof by microbial techniques, the steps of culturing, extraction and, if necessary, separation/purification are performed. Any strain may be used as long as it is a strain of *Bacillus subtilis* having growth inhibitory activity against the pathogen *Streptomyces* spp. *Bacillus subtilis* may be cultured in a manner well known to those skilled in the art. The compound of Formula I or a salt thereof may be extracted from the *Bacillus subtilis* culture, for example, through solvent extraction. By way of illustrative example, extraction may be accomplished by using ethyl acetate or a solvent which is slightly more polar than ethyl acetate and by using an apparatus allowing full mixing of the mixture. Separation/purification may be accomplished by using techniques well known to those skilled in the art, either alone or in combination, as exemplified by thin-layer chromatography (TLC), high performance liquid chromatography (HPLC), etc. The separated components can be confirmed for their structure by a combination of standard analysis means such as NMR, GC-MS and IR.

The antibacterial activity of a compound of Formula I or a salt thereof against actinomycetes of the genus *Streptomyces,* which are pathogens of potato scab, can be confirmed by test procedures well known to those skilled in the art. For example, pre-sterilized paper disks may be soaked with a peptide of surfactin type and then dried. These paper disks may be placed on the center of spore plates of *Streptomyces* spp., incubated for a given period of time and then measured for the radius of each inhibition circle to evaluate the antibacterial activity.

The composition of the present invention for controlling scab disease in agricultural products desirably comprises a compound of Formula I or a salt thereof in an amount of about 0.1 to 100 mg/kg, more preferably 1 to 10 mg/kg, as calculated on the basis of free form.

The composition of the present invention for controlling scab disease in agricultural products may comprise cells of *Bacillus subtilis,* preferably cells of *Bacillus subtilis* strain SAM2267 (FERM P-17761). These cells can be obtained by culturing *Bacillus subtilis* under conditions suitable for its growth and then collecting the cells. These cells may be either in a dry or a wet state. It should be noted that these cells may also be sprayed over the soil separately, i.e., not as an ingredient in the composition of the present invention for controlling scab disease in agricultural products.

The composition of the present invention for controlling scab disease in agricultural products may comprise an agricultural material. Examples of such a material include those which can be used for cultivation of agricultural products, such as soil, soil substitutes (e.g., vermiculite), soil conditioners (e.g., zeolite), fertilizers, organic materials (e.g., industrial food waste), and fermented products thereof (preferably aerobic fermented products), as well as combinations thereof. Among them, preferred are industrial food waste and zeolite.

Industrial food waste is not limited in any way as long as it is derived from source materials, product contents, production steps, waste fluids or the like, and can be restored to the soil either directly or after fermentation or other treatments. Examples of industrial food waste include saccharification residue or yeast wastes which are discharged from beer factorie, malt root (i.e., a malt-derived byproduct generated during malt preparation), grape pomace, activated charcoal and sludge, as well as combinations thereof, etc. Among them, extracted coffee residue and/or extracted tea residue are preferred examples. The term "extracted coffee residue" or "extracted tea residue" is intended to mean, for example, an extracted residue of coffee or teas generated in beverage production factories or food-service industries such as coffee shops, fast-food shops, etc. Such extracted coffee residue or extracted tea residue may be used either alone or in combination.

The composition of the present invention is particularly useful for controlling scab disease in beet, carrot, radish and potato, and more particularly useful for controlling potato scab.

### Method for controlling scab disease in agricultural products

In the method of the present invention for controlling scab disease in agricultural products, the scab disease is controlled by using a composition for controlling scab disease in agricultural products, which comprises a compound of Formula I or a salt thereof. This method comprises, for example, 1) a step where a composition for controlling scab disease in agricultural products, which comprises a compound of Formula I or a salt thereof, is contacted with seed potatoes, 2) a step where a composition for controlling scab disease in agricultural products, which comprises a compound of Formula I or a salt thereof, is sprayed over the plant body, or 3) a step where a composition for controlling scab disease in agricultural products, which comprises a compound of Formula I or a salt thereof, is mixed into or sprayed over the soil.

In the method where a composition for controlling scab disease in agricultural products, which comprises a compound of Formula I or a salt thereof, is mixed into or sprayed over the soil, the compound of Formula I or a salt thereof is preferably mixed into or sprayed over the soil in an amount of 0.001 to 50 mg/m², more preferably 0.01 to 5 mg/m² per unit area of soil, as calculated on the basis of free form. The method of the present invention ensures a continuous antibacterial effect throughout the cultivation period of agricultural products when the soil is treated once before starting cultivation of the agricultural products.

### Method for cultivating agricultural products

In the method of the present invention for cultivating an agricultural product, the agricultural product is cultivated while controlling scab disease by using a control composition comprising a compound of Formula I or a salt thereof. Scab disease control is achieved when 1) a composition for controlling scab disease in agricultural products, which comprises a compound of Formula I or a salt thereof, is contacted with seed potatoes, 2) a composition for controlling scab disease in agricultural products, which comprises a compound of Formula I or a salt thereof, is sprayed over the plant body, or 3) a composition for controlling scab disease in agricultural products, which comprises a compound of Formula I or a salt thereof, is mixed into or sprayed over the soil.

The present invention will now be described in more detail by way of the following experiments and examples, which are not intended to limit the scope of the present invention.

### EXAMPLES

### Example 1. Evaluation of antibacterial activity against potato scab pathogen: Effect of surfactin sodium concentrations in medium

An agar medium (containing 10 g soluble starch, 1 g sucrose, 1 g yeast extract, 0.1 g KH₂PO₄, 0.1 g NaNO₃, 0.1 g KCl, 0.1 g MgSO₄·7H₂O and 15 g agar in 1 L distilled water, adjusted to pH 7.0) was prepared to contain surfactin sodium (Wako Pure Chemical Industries, Ltd., Japan; which is also used in the subsequent examples) at a final concentration of 10 µg/ml, 50 µg/ml or 100 µg/ml. Another agar medium (whose composition is the same as above) free from surfactin sodium was also prepared for use as a control. Each agar medium was smeared with a spore suspension of the potato scab pathogen *Streptomyces turgidiscabies* 91SY-12 (hereinafter referred to as "the strain 91SY-12") and incubated at 30°C for 3 days. As a result, there was a tendency to inhibit spore formation at each tested concentration of surfactin sodium, i.e., 10 µg/ml, 50 µg/ml or 100 µg/ml (Figure 1).

### Example 2. Evaluation of antibacterial activity against potato scab pathogen (paper disk method): Effect of surfactin concentrations

The same agar medium as shown in Example 1 (being free from surfactin or surfactin sodium) was smeared with a spore suspension of the strain 91SY-12, and 4 paper disks (8 mm diameter) were placed thereon. A solution of surfactin in methanol was allowed to soak into the paper disks in a constant volume of 50 µl with varying surfactin concentrations: 0 mg/ml, 0.01 mg/ml, 0.1 mg/ml and 1.0 mg/ml. As a result of incubation at 30°C for 6 days, there was no inhibitory effect on spore formation for methanol alone and 0.01 mg/ml surfactin, whereas spore formation was inhibited, albeit weakly, at 0.1 mg/ml surfactin and inhibited strongly at 1.0 mg/ml surfactin (Figure 2).

### Example 3. Evaluation of antibacterial activity against potato scab pathogen (paper disk method): Effect of added surfactin volumes

In the same manner as shown in Example 2, the agar medium was smeared with a spore suspension of the strain 91SY-12, and 4 paper disks (8 mm diameter) were placed thereon. Surfactin was dissolved in methanol to give a concentration of 10 mg/ml, 10 µl, 50 µl or 100 µl of which was then allowed to soak into the paper disks. As a result of incubation at 30°C for 11 days, spore formation was strongly inhibited for each tested volume of surfactin (Figure 3).

### Example 4. Use of composition for controlling potato scab

Surfactin sodium (0.1 g) was dissolved in water (20 L) and mixed with zeolite (100 kg) to prepare a composition for controlling scab disease. This composition was sprayed over 10 ares of agricultural land, followed by planting potatoes. This agricultural land prevented scab disease from occurring and enabled the provision of good potatoes.

### Example 5. Use of composition for controlling potato scab

Extracted oolong tea residue, extracted coffee residue and sludge were used as source materials to prepare a compost. Surfactin sodium (0.1 g) was dissolved in water (30 L) and mixed with this compost (30 kg) and zeolite (70 kg) to give a composition for controlling scab disease. This composition was sprayed over 10 ares of agricultural land, followed by planting potatoes. This agricultural land prevented scab disease from occurring and enabled the provision of good potatoes.

### INDUSTRIAL APPLICABILITY

Although there are many reports of antibacterial compounds derived from *Bacillus subtilis* cultures, it had not been known that a compound of Formula I or a salt thereof inhibits the growth of *Streptomyces* spp., which are pathogens of scab disease, until the inventors of the present invention showed this finding. Above all, surfactin has been proven to be highly safe, as is also evident from the fact that it is widely used in facial cleansers, shampoos and the like. The present invention not only provides a promising means for controlling pathogens of scab disease in agricultural products including potato, but also contributes to the elucidation of antagonistic mechanisms against pathogens of scab disease, and so on.

## Claims

1. A composition for controlling scab disease in an agricultural product, which comprises a compound of Formula I or a salt thereof: [Formula 1] 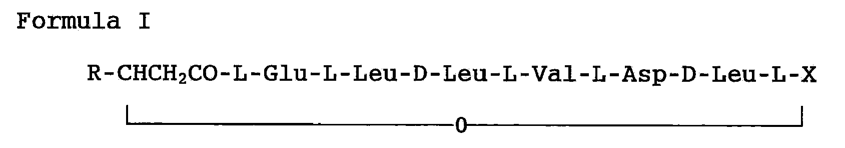 [wherein R is C₁₁-C₁₄ alkyl, and X is selected from Leu, Val or Ile].

2. The composition according to claim 1, wherein the compound of Formula I is surfactin.

3. The composition according to claim 1, which comprises the compound of Formula I or a salt thereof at a concentration of 1 to 10 mg/kg, as calculated on the basis of free form.

4. The composition according to claim 3, wherein the compound of Formula I is surfactin.

5. The composition according to any one of claims 1 to 4, wherein the scab disease in an agricultural product is potato scab.

6. The composition according to any one of claims 1 to 4, wherein the scab disease in an agricultural product is scab disease whose pathogen is an actinomycete of the genus *Streptomyces.*

7. The composition according to claim 6, wherein the actinomycete of the genus *Streptomyces* is *Streptomyces turgidiscabies.*

8. The composition according to any one of claims 1 to 7, which further comprises cells of *Bacillus subtilis.*

9. The composition according to any one of claims 1 to 8, which further comprises an agricultural material.

10. The composition according to claim 9, wherein the agricultural material is zeolite.

11. The composition according to claim 9, wherein the agricultural material is an industrial food waste.

12. The composition according to claim 11, wherein the industrial food waste is extracted coffee residue and/or extracted tea residue.

13. A method for controlling scab disease in an agricultural product, which comprises using the composition according to any one of claims 1 to 12.

14. The method according to claim 13, wherein the composition according to any one of claims 1 to 12 is contacted with seed potatoes.

15. The method according to claim 13, wherein the composition according to any one of claims 1 to 12 is sprayed over the plant body.

16. The method according to claim 13, wherein the composition according to any one of claims 1 to 12 is mixed into or sprayed over the soil.

17. The method according to claim 16, wherein the compound of Formula I or a salt thereof is mixed into or sprayed over the soil in an amount of 0.01 to 5 mg/m² per unit area of soil, as calculated on the basis of free form.

18. The use of a compound of Formula I or a salt thereof for growth inhibition of an actinomycete of the genus *Streptomyces,* which is a pathogen of scab disease in an agricultural product including potato.

19. A method for cultivating an agricultural product, which comprises using the composition according to any one of claims 1 to 12 during cultivation of the agricultural product.
